# EUROPEAN PATENT APPLICATION

(11) **EP 3 875 086 A1**
(43) Date of publication of application: **08.09.2021**
(21) Application number: 20160943.5
(22) Date of filing: 04.03.2020
(51) Int. Cl.: A61K 31/4178, A61K 31/422, A61P 43/00

(54) **DUAL-INHIBITORS OF CELLULAR NECROPTOSIS AND FERROPTOSIS FOR USE IN THE TREATMENT OF ORGAN TRANSPLANT PATIENTS**

(71) Applicant: Technische Universität Dresden, 01069 Dresden (DE); Rheinische Friedrich-Wilhelms-Universität Bonn, 53113 Bonn (DE)
(72) Inventor: LINKERMANN, Andreas, 01309 Dresden (DE); GÜTSCHOW, Michael, 53115 Bonn (DE); STEINEBACH, Christian., 53127 Bonn (DE)
(74) Representative: Hertin und Partner Rechts- und Patentanwälte PartG mbB

(57) **Abstract**

The invention relates to chemical compounds for use as a medicament in the prevention of organ transplant rejection and/or transplant organ damage. In embodiments of the invention, the compound inhibits and/or reduces ferroptosis and necroptosis of cells of the transplanted organ. In further embodiments, the patient is at risk of transplant rejection and/or is at risk of or shows signs of ischemia-reperfusion injury and/or necroinflammation.

## Description

The invention relates to chemical compound for use as a medicament in the prevention of organ transplant rejection and/or transplant organ damage. In embodiments of the invention, the compound inhibits and/or reduces ferroptosis and necroptosis of cells of the transplanted organ. In further embodiments, the patient is at risk of transplant rejection and/or is at risk of or shows signs of ischemia-reperfusion injury and/or necroinflammation.

### BACKGROUND OF THE INVENTION

The basic pathophysiological principle of various common diseases, e.g. heart attacks, strokes, poisoning, septic organ damage, but also tumor diseases, is the death of cells. In such diseases cell death is practically always associated with a breakdown of the cell membrane, which is called necrotic cell death or necrosis.

Transplantation is invariably associated with organ injury following donor organ procurement. Death of cells in the transplant organ can negatively impact organ function if sufficient numbers of parenchymal cells are eliminated and not replaced as part of remodeling. Additionally, by the release of cell contents, the necrotic cell death can alter immune responses that are related to ischemia-reperfusion injury and alloimmune rejection. There is increasing awareness of the link between innate and adaptive immunity, and the profound influence inflammation has on organ function, tolerance induction, rejection responses and perhaps survival. Unfortunately, long-term survival of transplants has not been greatly impacted by advances in transplant strategies that solely target recipient immune responses. The significance of necrotic cells death in the pathogenesis of solid organ injury and in particular in organ transplantation has been reviewed by Zhao et al (Cell Death and Disease (2015) 6, e1975; doi:10.1038/cddis.2015.316).

Necrosis is highly pro-inflammatory and until recently it has been assumed that necrosis is not genetically regulated. However, this hypothesis has been disproved in recent years. It is now understood that two intracellular signaling pathways are essentially responsible for regulating necrotic cell death. These pathways, necroptosis and ferroptosis, are mechanistically independent of each other and must be blocked in therapeutic combination in order to achieve an organo-protective effect.

However, to date no single inhibitor that can block both regulatory pathways has been identified. In contrast, it is only possible to use separate molecules or inhibitors for each of the two pathways. However, for regulatory approval of a drug/molecule, clinical trials including comprehensive safety assessment must be e carried out for each individual drug. In addition, the possible interactions with other drugs must be tested for each individual substance. Accordingly, the approval for a treatment using a dual-inhibitor strategy using different inhibitors for each of the two signaling pathways will be expensive and long process.

In light of the prior art there remains a significant need in the art to provide molecules or compounds that effectively block both pathways of necrotic cells death, necroptosis and ferroptosis, in order to avoid the need for multiple inhibitors that may negatively influence each other's function and require laborious and lengthy testing. Such drugs or molecules are in particular needed in the context of organ transplantation, where necrotic cells death in the transplanted organ is often associated with organ dysfunction and rejection in the recipients.

### SUMMARY OF THE INVENTION

In light of the prior art the technical problem underlying the present invention is to provide alternative and/or improved means for prevention of organ transplant rejection and/or transplant organ damage. In particular, the technical problem to be solved by the present invention can be defined as the provision of an inhibitor that simultaneously blocks ferroptosis and necroptosis.

This problem is solved by the features of the independent claims. Preferred embodiments of the present invention are provided by the dependent claims.

The invention therefore relates to a compound according to Formula I for use as a medicament in the prevention of organ transplant rejection and/or transplant organ damage, 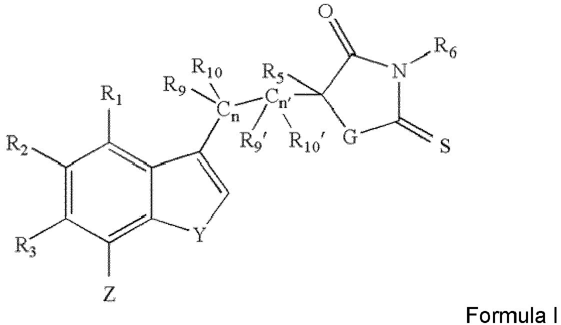 or a stereoisomeric form thereof, a pharmaceutically acceptable acid or base addition salt thereof; wherein
- Y is S or NR₈;
- G is O or NR₇, preferably NH;
- Z is Cl or OR₁₁;
- R₁, R₂, and R₃ are, independently of each other, H, OH, OR₈, F, CI, Br, I, N(R₈)₂, COOH, CO₂R₈, NO₂, NHC(O)R₈, lower alkyl, substituted lower alkyl, aryl, substituted aryl, heteroaryl, or substituted heteroaryl, preferably H;
- R₅, and R₇ are, independently of each other, H or lower alkyl, preferably H;
- R₈ is H, lower alkyl, substituted lower alkyl, aryl, substituted aryl, arylalkyl, alkenyl, alkynyl, heteroaryl, or substituted heteroaryl, preferably H;
- R₉, R₁₀, R₉', R₁₀', are, independently of each other, H, F, CI, Br, I, lower alkyl, substituted lower alkyl, or a three to six membered cycloalkyl or substituted cycloalkyl that includes Cₙ and/or Cₙ', preferably H;
- R₁₁ is alkyl, polyethylene or arylalkyl or a chemically divers linker structure with a terminal biotin moiety or any other terminal moiety able to bind to a functional protein; and
- n and n' are, independently of each other, an integer from zero to five, wherein the sum of n and n' is preferably one.

In embodiments, the invention therefore relates to a compound according to Formula II for use as a medicament in the prevention of organ transplant rejection and/or transplant organ damage, 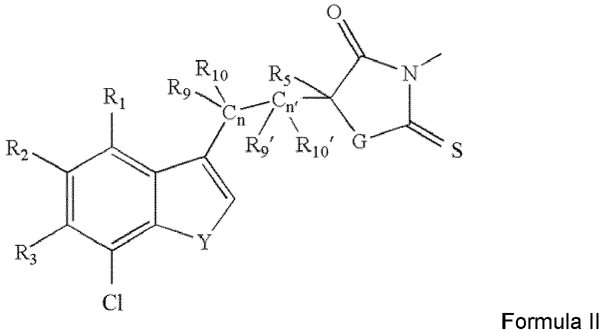 or a stereoisomeric form thereof, a pharmaceutically acceptable acid or base addition salt thereof; wherein
- Y is S or NR₈;
- G is O or NR₇, preferably NH;
- R₁, R₂, and R₃ are, independently of each other, H, OH, OR₈, F, Cl, Br, I, N(R₈)₂, COOH, CO₂R₈, NO₂, NHC(O)R₈, lower alkyl, substituted lower alkyl, aryl, substituted aryl, heteroaryl, or substituted heteroaryl, preferably H;
- R₅, and R₇ are, independently of each other, H or lower alkyl, preferably H;
- R₈ is H, lower alkyl, substituted lower alkyl, aryl, substituted aryl, arylalkyl, alkenyl, alkynyl, heteroaryl, or substituted heteroaryl, preferably H;
- R₉, R₁₀, R₉', R₁₀', are, independently of each other, H, F, CI, Br, I, lower alkyl, substituted lower alkyl, or a three to six membered cycloalkyl or substituted cycloalkyl that includes Cₙ and/or Cₙ', preferably H;
- n and n' are, independently of each other, an integer from zero to five, wherein the sum of n and n' is preferably one.

It was entirely unexpected that a compound according to formula I or II is effective in inhibiting both necroptotic as well as ferroptotic cell death pathways. This surprising double-inhibition was found to be strictly associated with the presence of three substituents, namely the Cl or alkyl, polyethylene or arylalkyl or a chemically divers linker structure with a terminal biotin moiety or any other terminal moiety able to bind to a functional protein at position 7 of the bicyclic indole structure, the methyl group bound to the N of the five-member heterocycle, as well as the S connected to the five-member heterocycle via a double-bond. It was surprisingly found that in absence of one of these three residues either the ferroptosis inhibitory activity or the necroptosis inhibitory activity or both activities were lost.

The newly identified technical effect of the compounds of the invention, namely previously unknown double-inhibition of the two cell death pathways necroptosis and the ferroptosis, leads to the opening of a new field of application of the compounds, namely in the treatment of prevention of conditions that are associated with the induction of necroptotic and ferroptotic cell death mechanisms, such as ischemia associated malignancies. Furthermore, a new sub-group of patients that are at risk of suffering or do already suffer from a condition that involves both ferroptosis and necroptosis can now be treated by means of a single compound of the invention instead of using two distinct inhibitors for each pathway.

The compounds described herein were found to be particularly useful in the prevention of organ transplant rejection and/or the prevention of transplant organ damage. The compounds of the invention can be administered to the transplant organ, and/or they can be administered to the patient receiving the organ transplant. In embodiments the compound can also be administered to the organ donor before removal of the organ. In that way the inhibitor is already present in the tissue before the organ is removed from the blood circulation of the donor. The inhibitor can also be present in the buffer or medium that is used to maintain the transplant organ after removal from the donor. Additionally, it may be useful to administer the compound to the recipient, before transplantation, at the time point of transplantation and/or after transplantation. In embodiments of the invention, the transplant organ is exposed to the compound during the whole process of transplant organ procurement, transport, storage, transplantation and during the initial phase after transplantation to the recipient.

The advantage of the present invention is that it is possible to inhibit both pathways leading to necrotic cell death, namely ferroptosis and necroptosis by administration of an effective amount of a single compound. The inhibition of these pathways significantly reduces the number of necrotic cells in the transplant organ at the time point of and/or after transplantation, which prevents the exposure of intracellular structures and antigens derived from the donor tissue to the immune system of the recipient. This is particularly advantageous since such intracellular antigens may lead to an activation of the recipient's immune system. Such an activation may contribute to a rejection of the transplant organ or the induction of mechanisms that negatively influence function and/or survival of the donor organ in the recipient.

In embodiments, the compound inhibits and/or reduces ferroptosis and necroptosis of cells of the transplanted organ. The present invention is based on the unexpected finding that the compounds of the invention can inhibit and/or reduce activation of cellular ferroptosis and necroptosis pathways, leading to the rupture of cell membranes and the exposure of intracellular material to the extracellular space.

In embodiments, the compound of the invention is administered to the patient and/or the organ within the first 10 days after, preferably 3 days after, more preferably at the time point of or before organ transplantation, such as at the time point of organ removal from the donor.

In embodiments of the invention, in order to prevent transplant organ damage and/or rejection, the compounds of the invention are administered to the patient receiving the transplant organ. Administration to the receiving patient can occur before transplantation of the organ in order to ensure that the inhibitor is already present in the recipients system in sufficient amounts to inhibit the necrosis pathways as soon as the transplant organ is introduced in the recipients body and/or is connected to the recipients circulatory system.

Furthermore, the recipient can receive the compound at the time point of transplantation and/or during the process of transplanting the organ. Administration can also occur after transplantation of the organ, either as a maintenance of a previous administration or exposure of the organ, or as an initial exposure. Administration to the patient can occur on the day of transplantation, and/or 1, 2, 3, 4, 5, 6, 7, 8, 9, 10, 11, 12, 13, 14, 16, 18, 20, 254, 28, 30, 35, 40, 45 or 50 days after transplantation.

In embodiments, the compound for use as a medicament of the invention is administered to the patient in advance of organ transplantation in a prophylactic treatment for the prevention of organ rejection.

Furthermore, the compounds of the invention can be administered at any time point after transplantation upon occurrence of a suspicion that the patient may be at risk of developing an organ rejection reaction or organ damage.

In embodiments, the compounds of the invention are administered to the organ before it is transplanted to the receiving patients. In embodiments, the compound is administered to the organ donor before procurement of the organ from the donor's body. This embodiment is particularly advantageous since the inhibitors are can already be delivery to the organ before it is removed from circulation at before a risk of induction of necrotic signaling pathways is present. Furthermore, the organ can be exposed to the compounds of the invention as soon as it is removed from the donor's body, for example by supplementing the medium or buffer solution used for storage and potentially perfusion of the organ outside the body. The exposure can be maintained until the organ is transplanted to the recipient patient, which may already have the compound in his system or who can receive the compound as soon as the organ is introduced into his system.

In further embodiments of the compound for use as a medicament as disclosed herein, the patient is at risk of transplant rejection, preferably acute transplant rejection. The risk of transplant rejection can be increased in certain individuals that are genetically predisposed, for example be the presence of certain genetic modification such as single nucleotide polymorphisms associated with an increased risk of transplant rejection. Further risk factors that may identify a patient as being at risk of transplant rejection can be the presence or identification of high panel reactive antibody (PRA), retransplant and deceased donor (DD) grafts, and/or (unrecognized) donor-specific antibody (DSA). These risk factors may be further modulated by epitope spreading.

Further risk factors or symptoms that define a patient as being at risk of transplant rejection, preferably acute transplant rejection, include individual immunosuppression metabolism and patient adherence.

In further embodiments, the patient is at risk or shows signs of acute transplant dysfunction. Acute transplant dysfunction can be caused by ischemic damage or immunological injury leading to serious consequences both in the short and long term. Biomarkers indicating transplant dysfunction are known to the skilled person and include serum creatinine concentrations. Further markers that can be measured even more early on before the actual manifestation of a dysfunction, in particular in case of a kidney transplant, include Neutrophil Gelatinase-Associated Lipocalin (NGAL) and Cystatin C and a urine panel including NGAL, II-18 and Kidney Injury Molecule 1 (KIM-1). Other markers are currently under investigation, including NephroCheck and serum concentrations of proenkephalin.

Furthermore, in the context of the invention the patient may be at risk of or may show signs of ischemia-reperfusion injury and/or necroinflammation. Such transplant patients are in acute need of inhibition of necrotic cell death and that therefore particularly profit from a compound of the invention inhibiting both regulatory pathways leading to necrosis.

Ischemia-reperfusion injury (IRI), also called reperfusion injury or reoxygenation injury, is the tissue damage caused when blood supply returns to tissue (re- + perfusion) after a period of ischemia or lack of oxygen (anoxia or hypoxia). The absence of oxygen and nutrients from blood during the ischemic period creates a condition in which the restoration of circulation results in inflammation and oxidative damage through the induction of oxidative stress rather than (or along with) restoration of normal function. Accordingly, IRI is a likely complication occurring upon organ transplantation, in particular if the organ was deprived from oxygen and/or other nutrients before transplantation. If such a deprivation is known to the treating medical personnel involved in the organ transplantation process, it is clear that the recipient is at risk of ischemia-reperfusion injury. Symptoms such as nausea, fatigue, lassitude, listlessness, vomiting, pruritus, itchiness and loss of consciousness are symptoms of uremia, the clinical consequence of ischemia-reperfusion injury and acute tubular necrosis.

Necroinflammation is an inflammatory process that is initiated be necrotic cell death leading to the exposure of normally intracellular components to the extracellular space. Necrotic type cell death pathways, including necroptosis and ferroptosis, inevitably release so-called intracellular damage-associated molecular patterns (DAMPs) leading to the activation of immune mechanisms causing inflammation. Signs or symptoms of a transplant patient that is at risk of or shows signs of necroinflammation include usual signs of inflammation that occur in the transplanted organ and connected or associated physiological systems. The systemic elevation of lactate dehydrogenase indicates necrosis in tissues and indirectly demonstrates necroinflammation. In addition, biopsies obtained from kidney transplants can ultimately prove tissue inflammation as a consequence of necrosis.

In embodiments of the invention, the compound is administered in combination with immunosuppression. In embodiments, the compound of the invention is administered in combination with other, such as known and established treatment measures for preventing and/or treating transplant organ dysfunction and/or rejection.

For example, the compounds of the invention may be administered to a patient that has received a transplant organ in combination with or in parallel to an immunosuppressive treatment, such as administration of immunosuppressive drugs, including, without limitation, corticosteroids, Prednisolone, Hydrocortisone, Calcineurin inhibitors, Ciclosporin, Tacrolimus, Anti-proliferatives, Azathioprine, Mycophenolic acid, mTOR inhibitors, Sirolimus, Everolimus. Furthermore, antibody-based treatments may be carried out as immunosuppressive therapy. Possible antibody-drugs include, without limitation, monoclonal anti-IL-2Ra receptor antibodies such as Basiliximab and Daclizumab, Polyclonal anti-T-cell antibodies such as Anti-thymocyte globulin (ATG) and Antilymphocyte globulin (ALG), Monoclonal anti-CD20 antibodies, such as Rituximab. Further treatment options that can be carried out in combination with or in parallel to the administration of the compounds of the invention include blood transfer, phtopheresis, extracorporeal phtoimmune therapy (ECP); bone marrow transplantation, which may lead to a replacement of the recipient's immune system; gene therapy, wherein for example the genes causing the rejection reaction may be inactivated.

In further embodiments of the invention, the patient is a kidney transplant patient.

In embodiments, the transplant organ is selected from the group comprising kidney, liver, heart, lung, pancreas, pancreatic islets, small intestine, and esophagus.In embodiments, the transplant organ is kidney. In embodiments, the transplant organ is liver. In embodiments, the transplant organ is heart. In embodiments, the transplant organ is lung. In embodiments, the transplant organ is pancreas. In embodiments, the transplant organ is pancreatic islets. In embodiments, the transplant organ is small intestine. In embodiments, the transplant organ is esophagus.

The invention is not limited to solid organ transplants, but also relates to bone marrow transplantation or transplantation of cells. In the context of the invention, pancreatic islets are regarded as organs. However, the present invention also relates to transplantation of cells, such as specific stem cells or parenchymal cells that have been recovered from a donor or from a laboratory.

Furthermore, in embodiments of the invention the compound inhibits the kinase activity of Receptor-interacting serine/threonine-protein kinase 1 (RIPK1) and lipid peroxidation. It was entirely surprising that compound of the present invention can simultaneously inhibit RIPK1 and lipid peroxidation in cells and therefore can simultaneously prevent necrotic cell death via inhibition of both necroptosis and ferroptosis.

This is an important advantage of the invention, since inhibition of only one of the pathways may result in compensation by activation of the other. Accordingly, inhibition of both pathways is required. Importantly, it is great advantage that the invention enables this by means of only a single kind of compound. Accordingly, it is not necessary to perform laborious and time-consuming studies studying double-inhibitor strategies.

There are several suitable assays for measuring RIPK1 activity and lipid peroxidation and for testing the ability of a compound to inhibit these processes that are known to a skilled person or can be identified by a skilled person. Western Blot analysis of phosphorylation of RIPK3 or MLKL and direct detection of phosphorylation of RIPK1 by monoclonal antibodies are available. There are also ready to use products for such purposes available, such as RIPK1 Kinase Assay Kit from BPS Bioscience, Inc. (Cat-No. #79560), or the RIPK1 Kinase Enzyme System available form PROMEGA. Similarly, Lipid peroxidation kits are available from Abcam (ab118970), BioTrend (MBS822354-100), ThermoFisher (Imag-iT™; C10445), Sigma-Aldrich (PeroxiDetect™ Kit; PD1-1KT).

In embodiments, the compound of the invention inhibits and/or reduces exposure of intracellular antigens of the transplanted organ. It is a great advantage of the present invention that the number of cells dying through necrotic cell death in the process of organ procurement, storage and transplantation can be significantly reduced, leading a reduced exposure of intracellular antigens or DAMPS from the cells of the transplant. Accordingly, the compounds of the invention can reduce the risk of activation or over-activation of the recipient's immune system by the transplant, the transplanted cells or intracellular components of the cells of the transplanted organ.

Also, the compound for use as a medicament according to the present invention can inhibit and/or reduce activation of the patient's immune system by intracellular antigens of the transplanted organ (necroinflammation). Furthermore, also the activation of the patient's immune system by intracellular DAMPS of the transplant can be reduced.

In certain embodiments of the invention, administration of the compound, preferably intravenously, enhances function of the transplanted organ in the patient. A further beneficial effect of the invention is that by preventing or inhibiting necrotic cells death in the transplant organs it is possible to maintain a larger number of living and functional cells in the transplant, leading to an improved function of the organ upon transplantation into the recipient patient.

In specific embodiments of the invention, the compound is Nec-1f,

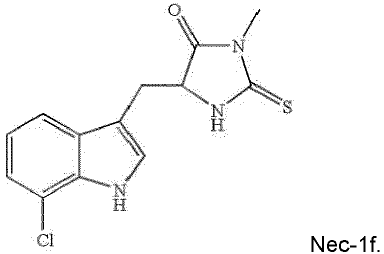

It was surprisingly found that the compound designated as Nec-1f is particularly advantageous and shows very strong inhibitory activity for both pathways leading to necrotic cell days, namely ferroptosis and apoptosis. It was entirely unexpected that this specific compound can effectively inhibit both signaling pathways and therefore represents a preferred compound of the invention. Nec-1f has a molecular weight of 293,88 g/mol.

The invention further relates to pharmaceutical compositions comprising a compound of the present invention according to formula I or formula II, preferably Nec-1f, for the prevention of organ transplant rejection and/or transplant organ damage.

### DETAILED DESCRIPTION OF THE INVENTION

All cited documents of the patent and non-patent literature are hereby incorporated by reference in their entirety.

The present invention is directed to a compound as disclosed herein such as a compound according to formula I or formula II, for use as a medicament in the prevention of organ transplant rejection and/or transplant organ damage.

As used herein, the term "organ transplant rejection" or "transplant rejection" relates to any pathophysiological mechanism of an organism that leads to the rejection or destruction of a transplanted organ or tissue or of transplanted cells. In the context of the invention, the term "transplant organ" refers to solid organ transplants, but also to tissue transplantation, (stem) cell transplantation or the like. Comprise by the term organ transplant are in particular also bone marrow transplants, blood transfusions, stem cell transplantation, cartilage or other tissue transplantation, which may originate not directly from a donor's body, but from a laboratory preparation. Furthermore, comprises a classical organ transplants, such as pancreas, pancreatic islets, liver, lung, heart, kidney, small intestine, intestine, esophagus, skin and further organs and further organ transplants known in the art and by the skilled person. In embodiments of the invention, the transplant can be a xenotransplant organ.

Transplant rejection occurs for example when transplanted tissue is rejected by the recipient's immune system, which attacks and eventually destroys the transplanted tissue. Transplant rejection can be lessened by determining the molecular similitude between donor and recipient and by use of immunosuppressant drugs after transplant. The risk of transplant rejection can be reduced by using approaches that prevent an activation of the immune system by the transplanted tissue, as is the case in the context of the present invention.

Rejection is an adaptive immune response via cellular immunity (mainly mediated by killer T cells inducing apoptosis of target cells) as well as humoral immunity (mediated by activated B cells secreting antibody molecules), though the action is joined by components of innate immune response (phagocytes and soluble immune proteins). Different types of transplanted tissues tend to favor different balances of rejection mechanisms. The activation of the adaptive immune response in rejection reactions can be modulated by components and mechanisms that are attributed to the innate immune system.

An animal's exposure to the antigens of a different member of the same or similar species is allostimulation, and the tissue is allogenic. Transplanted organs are often acquired from a dead person (usually a host who had succumbed to trauma), whose tissues had already sustained ischemia or inflammation. Dendritic cells (DCs), which are the primary antigen-presenting cells (APCs), of the donor tissue migrate to the recipient's peripheral lymphoid tissue (lymphoid follicles and lymph nodes) and present the donor's self-peptides to the recipient's lymphocytes (immune cells residing in lymphoid tissues). Lymphocytes include two classes that enact adaptive immunity, also called specific immunity: Lymphocytes of specific immunity, in particular T cells, including the subclasses helper T cells and killer T cells, and B cells. The recipient's helper T cells coordinate specific immunity directed at the donor's self-peptides or at the donor's Major histocompatibility complex molecules, or at both.

Transplant rejection can be classified as hyperacute, acute, or chronic. Hyperacute rejection is usually caused by specific antibodies against the graft and occurs within minutes or hours after grafting. Acute rejection occurs days or weeks after transplantation and can be caused by specific lymphocytes in the recipient that recognize HLA antigens in the tissue or organ grafted. Finally, chronic rejection usually occurs months or years after organ or tissue transplantation.

Initiated by preexisting humoral immunity, *hyperacute* rejection can manifest within minutes after transplantation, and if tissue is left implanted brings systemic inflammatory response syndrome. Of high risk in kidney transplants is rapid clumping, namely agglutination, of red blood cells (RBCs or erythrocytes), as an antibody molecule binds multiple target cells at once.

While kidneys can routinely be obtained from human donors, most organs are in short supply leading to consideration of xenotransplants from other species. Pigs are especially likely sources for xenotransplants, chosen for the anatomical and physiological characteristics they share with humans.

"Acute transplant rejection" is a process that is initiated upon the formation of cellular immunity against the transplant. Acute transplant rejection occurs to some degree in all transplants, except between identical twins, unless immunosuppression is achieved (usually through drugs). Acute rejection begins as early as one week after transplantation, the risk being highest in the first three months, though it can occur months to years later. Highly vascular tissues such as kidney or liver often host the earliest signs - particularly at endothelial cells lining blood vessels - though it eventually occurs in roughly 10 to 30% of liver transplants, and 10 to 20% of kidney transplants. A single episode of acute rejection can be recognized and promptly treated, usually preventing organ failure, but recurrent episodes lead to chronic rejection. It is believed that the process of acute rejection is mediated by the cell mediated pathway, specifically by mononuclear macrophages and T-lymphocytes. Histology of acute rejection is defined by dense lymphocytic cellular infiltrate as well as vasculitis of organ donor vessels.

As used herein, the term "chronic rejection" comprises a process described as long-term loss of function in transplanted organs via fibrosis of the transplanted tissue's blood vessels, which is also referred to as chronic allograft vasculopathy in the art, and chronic rejection due to other aspects of immunity.

The etiology of chronic rejection is incompletely understood. It was reported that a vital determinant of the complication of chronic solid organ allografts is by a vascular disease component of the transplanted graft.

In cardiac allograft vasculopathy, histologic evidence suggests that accumulation and activation of phagocytes can be a contributing factor. Phagocytic cells such as macrophages, monocytes, and immature dendritic cell subsets are found in this type of chronic cardiac transplant rejection. Furthermore, myeloid phagocytes crosstalk with B and T lymphocytes while also signaling and activating vascular smooth muscle cells and fibroblasts, and these can cause fibrous intimal thickening.

Chronic renal allograft rejection has been labeled as interstitial fibrosis and tubular atrophy reflecting the underlying histology and etiology. Chronic rejection is now cited as the leading cause of graft rejection.

Chronic allograft rejection can be caused by antibody-dependent complement activation lesions as well as cell arteritis leading to the development of interstitial fibrosis/tubular atrophy (IF/TA). This injury can appear early after transplantation. At 1-year post-transplant, greater than 81% of the kidneys have minimal lesions of IF/TA that tend to progress over time; these lesions affect greater than 50% of transplanted kidneys with severe lesions at five years. Other proposed pathophysiologic mechanisms include: Calcineurin inhibitors (CNI) toxicity, Chronic dysfunction of the solid organ causing premature graft loss, alloimmunization to minor HLA antigens, Production of interferon-gamma and IL-2 by Th1 lymphocytes, Secretion of cytokines by endothelial cells.

In the context of the invention, the term "transplant organ damage" relates to any kind of damage, such as cell death within the transplant that is not physiological. The cause of the damage can have multiple reasons, basically anything a skilled person can envision, ranging from immunological damage caused by a reaction of the host's immune system, mechanical or toxic injury or damage that could still have occurred in the donor's body or during the transport of the organ from the site of recovery to the site of transplantation.

As used herein, a patient or subject "at risk of' a clinical condition, such as organ transplant rejection or ischemia-reperfusion injury, is a subjection who does not (yet) suffer from the respective condition, or at least not to its full extend. The subject may however show initial signs that can be associated with early stages of the respective condition, or displays risk factors associated with the respective condition, such as the occurrence of specific biomarkers, genetic predisposition, behavioral risk factors, medical history, or any other factor known by a skilled person and that is indicative that the person has an increased risk of developing the respective condition.

Similarly, as understood in the context of the present invention a person, subject or patient "showing signs of acute transplant dysfunction" is someone who displays initial symptoms, risk factors, biomarkers, genetic or behavioral predispositions, such as a certain medical history, that can be indicative or associated with a higher risk of developing the respective condition.

In the context of the invention, the term "ischemia-reperfusion injury" (IRI), sometimes also referred to as reperfusion injury or reoxygenation injury, is the tissue damage caused when blood supply returns to tissue after a period of hypoperfusion or lack of oxygen. The absence of oxygen and nutrients from blood during the ischemic period creates an intracellular lactate acidosis, a condition in which the restoration of circulation results in necrotic cell death and necroinflammation. This results in oxidative damage through the induction of oxidative stress rather than (or along with) restoration of normal function. Accordingly, IRI is a likely complication occurring upon organ transplantation, in particular if the organ was deprived from oxygen and/or other nutrients before transplantation. If such a deprivation is known to the treating medical personnel involved in the organ transplantation process, it is clear that the recipient is at risk of ischemia-reperfusion injury. Reperfusion of ischemic tissues is often associated with microvascular injury, particularly due to increased permeability of capillaries and arterioles that lead to an increase of diffusion and fluid filtration across the tissues. Activated endothelial cells produce more reactive oxygen species but less nitric oxide following reperfusion, and the imbalance results in a subsequent inflammatory response. The inflammatory response is partially responsible for the damage of reperfusion injury. White blood cells, carried to the area by the newly returning blood, release a host of inflammatory factors such as interleukins as well as free radicals in response to tissue damage. The restored blood flow reintroduces oxygen within cells that damages cellular proteins, DNA, and the plasma membrane. Damage to the cell's membrane may in turn cause the release of more free radicals. Such reactive species may also act indirectly in redox signaling to turn on apoptosis. White blood cells may also bind to the endothelium of small capillaries, obstructing them and leading to more ischemia.

IRI occurs in a wide range of organs including the heart, lung, kidney, gut, skeletal muscle and brain and may involve not only the ischemic organ itself but may also induce systemic damage to distant organs, potentially leading to multi-system organ failure. IRI is a significant challenge faced by physicians in transplantation medicine, in particular in the case of kidney transplantation, and is an important risk factor associated with acute organ failure, such as acute kidney injury after transplantation.

As used herein, the term necrosis refers to a specific class of cell death mechanisms involving the rupture or breakdown of the plasma membrane of the dying cell, leading to the exposure of intracellular components to the extracellular space. As used herein, the term necrosis is understood a "regulated necrosis", which is a genetically determined process that results in plasma membrane rupture and the release of intracellular content, comprising so-called damage associated molecular patterns (DAMPs). Pathways of regulated necrosis include but are not limited to necroptosis and ferroptosis. Necroptosis and ferroptosis are independent signaling pathways that contribute to organ damage, which is often summarized as necrotic cells death or necrotic organ/tissue damage.

The signaling pathway of necroptosis involves phosphorylated mixed linage kinase domain like (pMLKL), which is the only known mediator of necroptosis. pMLKL detection defines the activation of this pathway. Potentially, several kinases may phosphorylate MLKL, but only receptor interacting protein kinase 3 (RIPK3) has been described until now. MLKL carries several phosphorylation sites, and RIPK3 phosphorylates the so-called "activation loop". However, complete function of pMLKL requires the depohsphorylation at the hinge region that unleashes the deadly activity of a 4 helical bundle (4-HB) which binds PIP2 in the plasma membrane and tends to oligomerize with other pMLKL molecules. Downstream of pMLKL, sensitivity of cells to undergo necroptosis is controlled by proteins that control membrane blebbing and microvesical formation. Unlike previously suggested, pMLKL does not directly form pores in the plasma membrane of cells. Full activation of RIPK3 requires the assembly of the necrosome, a higher order structure that consists of oligomerized RIPK3 molecules that are stabilized by HSP90 and CDC37, two chaperones the loss of which results in defective necroptosis. Several triggers result in the formation of the necrosome. Death receptor (DR), e.g. TNFR1-stimulation in the presence of a caspase-inhibitor or a dysfunctional caspase-8 represents the most prominent and best investigated stimulus that requires RIPK1 kinase activity for necrosome formation. RIPK1, as RIPK3, contains a rip homotypic interacting motif (RHIM)-domain that intercalates with the RHIM of RIPK3 and prevents necrosome assembly. Inhibitors of RIPK1 kinase activity, such as Nec-1s and ponatinib, maintain the inactive state of RIPK3 potentially by keeping RHIM-RHIM-interactions intact. Necrosome assembly has been repeatedly reported downstream of Toll like receptors that bind to the intracellular adapter protein TRIF which also contains a RHIM domain and activation of this pathway results in robust RIPK1-independent necrosome formation.

In vivo, reperfusion following ischemic injury severely triggers necroptosis, and several other models have been described, such as injection of recombinant human TNFα into mice. In addition, protein kinase R (PKR) and DAI, a protein that is capable of but functionally not limited to viral DNA sensing activation as triggered by interferons, can activate RIPK1-mediated necrosome formation, but the relative contribution of these two factors remains unclear. Certainly, DAI robustly triggeres necrosome formation via its RHIM domain, possibly via nuclear signalling. However, importantly, necroptosis contributes to acute kidney injury in some models, such as ischemia, but inhibition of necroptosis does not affect other models, such as foliac acid induced AKI.

In contrast, during the signaling pathway of ferroptosis, peroxidation of membrane lipids, predominantly phosphatidylinositole and phosphotidylethanolamine, represents the point of no return resulting in loss of NADPH-abundance and synchronized regulated necrosis (SRN) in sensitive organs, such as the renal tubular compartment or the myocardium. Lipoxygenases (ALOX) mediate lipid peroxidation and are kept in their inactive state by the constitutively active function of glutathione peroxidase 4 (GPX4), a selenoenzyme that requires glutathione (GSH) to function. Ferroptosis may be triggered by inhibition of system Xc-minus, a cys/glu-antiporter in the plasma membrane by a lethal compound referred to as erastin. Inhibition of system Xc-minus functionally inhibits the activation of the GSH-synthase (GSSG), resulting in GSH depletion, dysfunction of GPX4 and ferroptosis. RSL3 induces ferroptosis directly by inhibition of GPX4, and certain nanoparticles are capable of inducing ALOX activation in vitro. Before lipid peroxidation occurs, it may be prevented by the ferrostatins liproxstatin, ferrostatin-1 (Fer-1), necrostatin-1 (Nec-1), and the novel compounds 16-86, XJB-5-131 and JP4-039. The latter carries intrinsic anti-necroptotic activity beyond its function as a ferrostatin.

Ferroptosis accounts for the majority of tubular cell loss during acute kidney injury because of its mechanism of SRN that shuts down an entire functional unit, but ferroptosis may be triggered by any cell death that occurs in cells of the functional syncytium because of the preterminal loss of NADPH that occurs in all cell death pathways.

Importantly, ferroptosis and necroptosis are both detrimental pathways involved in mediating necrotic cell death in transplant organs leading to necroinflammation which can promote transplant organ rejection.

In the context of the invention, the term necroinflammation relates to an inflammatory process caused by necrotic cell death, which may be mediated by ferroptosis or necroptosis, leading to the induction of inflammation. Two paramount features are associated with necrotic cellular damage. First, the cell loses its function. Second, the necrotic cell's environment gains access to previously hidden surfaces. Necrosis, therefore, is likely to activate resident immune cells. Upon additional production of pro-/anti-inflammatory cytokines, tissue hormones, and lipid peroxides in addition to the unregulated release of subcellular structures, such as broken mitochondria, peroxisomes, endoplasmic reticulum, nuclei etc., specific necrotic signaling pathways may modulate the immune response. Diseases associated with necroptosis and ferroptosis include acute myocardial infarction, stroke, acute kidney injury, acute liver failure, every solid organ transplant, pancreatitis, sepsis, and every severe intoxication.

As used herein, a compound that inhibits and/or reduces necroptosis and ferroptosis is a compound that significantly reduces the rate of these necrotic cell death mechanisms in a system exposed or treated to such a compound in comparison to an identical system that has not been exposed or treated by the respective compound. Inhibition of the necroptosis or ferroptosis pathways in an organism, such as a human or mammal, in particular in the context of transplantation of an allograft or xenograft, also reduces the activation of the immune system by intracellular antigens or DAMPS. The person skilled in the art knows how to determine the rate of necrotic cell death and can differentiate between different signaling pathways. This is evident from the different signaling pathways described above, whose activation can be routinely measured by a skilled person. Suitable methods have been disclosed in the art, for example in (Tonnus et al., J Pathol. 2019 Apr;247(5):697-707; PMID 30714148; and Weinlich et al., Nat Rev Mol Cell Biol. 2017 Feb;18(2):127-136; PMID 27999438).

As used herein, the terms "subject" and "patient" includes both human and veterinary subjects, in particularly mammals, and other organisms. The term "recipient" relates to a patient or subject that receives an organ transplant.

In the context of the present invention, the time point of organ removal is understood as the time point when an organ is removed from a donor's body, in particular the time point of disconnection from the donor circulation. In case of transplants that are derived from a laboratory situation, the time point of organ removal is the time point when the graft/tissue/organ/cells are removed from their usual environment for maintenance in the laboratory and transferred to a storage or transport environment.

As used herein "prevention" of organ transplant rejection and/or transplant organ damage is understood as relating to any method, process or action that is directed towards ensuring that a transplanted organ will not be terminally rejected by the organ recipient. Prevention relates to a prophylactic treatment intended to avoid a situation where a transplanted organ is rejected by the recipient. A "prophylactic" treatment is a treatment administered to a subject who does not exhibit signs of a disease or exhibits only early signs for the purpose of decreasing the risk of developing pathology.

The term "treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease.

In another aspect, the present invention provides pharmaceutically acceptable compositions, which comprise a therapeutically effective amount of one or more of the compounds described herein, formulated together with one or more pharmaceutically acceptable carriers (additives) and/or diluents. As described in detail, the pharmaceutical compositions of the present invention may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin; intravaginally or intrarectally, for example, as a pessary, cream or foam; sublingually; ocularly; transdermally; or nasally, pulmonary and to other mucosal surfaces.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations. Examples of such formulations include, but are not limited to DMSO, 10 mM DMSO, 8% hydroxypropyl-beta-cyclodextrin in PBS, propylene glycol, etc.

For example, in a certain embodiment the compounds of the invention, such as 893-54 can be used as 4 mM solution in 8% hydroxypropyl-beta-cyclodextrin in PBS for parenteral administration. In another embodiment 893-54 (0.40 mg/mL) was formulated as follows: 5.975 mg of 893-54 was weighed into a pre-cleaned 20 mL glass vial, 1.49 mL of propylene glycol was added to the vial followed by vortexing and sonication for 15 minutes, and 13.41 mL of sterile normal saline was added for a total volume of 14.9 mL followed by vortexing.

As set out herein, certain embodiments of the present compounds may contain a basic functional group, such as amino or alkylamino, and are, thus, capable of forming pharmaceutically acceptable salts with pharmaceutically-acceptable acids. The term "pharmaceutically-acceptable salts" in this respect refers to the relatively non-toxic, inorganic and organic acid addition salts of compounds of the present invention. These salts can be prepared in situ in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19.)

The pharmaceutically acceptable salts of the subject compounds include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically acceptable salts with pharmaceutically acceptable bases. The term "pharmaceutically-acceptable salts" in these instances refers to the relatively non-toxic, inorganic and organic base addition salts of compounds of the present invention. These salts can likewise be prepared in situ in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions. Examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, this amount will range from about 1% to about 99% of active ingredient, preferably from about 5% to about 70%, most preferably from about 10% to about 30%.

In certain embodiments, a formulation of the present invention comprises an excipient selected from the group consisting of cyclodextrins, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol, glycerol monostearate, and non-ionic surfactants; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made in a suitable machine in which a mixture of the powdered compound is moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Dissolving or dispersing the compound in the proper medium can make such dosage forms. Absorption enhancers can also be used to increase the flux of the compound across the skin. Either providing a rate controlling membrane or dispersing the compound in a polymer matrix or gel can control the rate of such flux.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenyl sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

In another aspect, the present invention relates to a method of treating a disease associated with cellular necrosis. In particular, the invention provides methods for preventing or treating a disorder associated with cellular necrosis in a mammal, comprising the step of administering to said mammal a therapeutically effective amount of a compound or therapeutic preparation of the present invention. In certain embodiments, the disorder associated with cellular necrosis is a neurological disorder such as trauma, ischemia or stroke. In other embodiments, the neurological disorder is a neurodegenerative disease, such as Parkinson's disease (PD), Alzheimer's disease (AD), amyotrophic lateral sclerosis (ALS), Huntington's disease (HD), and HIV-associated dementia (HAD). In other embodiments the disorder is an ischemic disease of organs including but not limited to brain, heart, kidney, and liver. In certain embodiments, the mammal is a primate, canine or feline subject. In other embodiments, the mammal is a human subject.

The phrase "therapeutically-effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment. The therapeutically effective amount or diagnostically effective amount of an agent will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects.

In general, a suitable daily dose of a compound of the invention will be that amount of the compound that is the lowest dose effective to produce a therapeutic effect. Such an effective dose will generally depend upon the factors described herein. Generally, doses of the compounds of this invention for a patient, when used for the indicated effects, will range from about 0.0001 to about 100 mg per kg of body weight per day. A non-limiting further ranges for a therapeutically effective amount, preferably daily amounts, of a compound and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

In certain embodiments, a compound or pharmaceutical preparation is administered orally. In other embodiments, the compound or pharmaceutical preparation is administered intravenously. Alternative routes of administration include sublingual, intramuscular, and transdermal administrations.

In certain embodiments, the present invention relates to ligands for inhibiting cell death, wherein the ligands are represented by any of the structures outlined above, and any sets of definitions associated with one of those structures. In certain embodiments, the ligands of the present invention are inhibitors of cell death. In any event, the ligands of the present invention preferably exert their effect on inhibiting cell death at a concentration less than about 50 micromolar, more preferably at a concentration less than about 10 micromolar, and most preferably at a concentration less than 1 micromolar.

The compounds of the invention can be tested in standard animal models of stroke and standard protocols such as described by Hara, H., et al. Proc Natl Acad Sci U S A, 1997. 94(5): 2007-12.

When the compounds of the present invention are administered as pharmaceuticals, to humans and animals, they can be given per se or as a pharmaceutical composition containing, for example, 0.1% to 99.5% (more preferably, 0.5% to 90%) of active ingredient in combination with a pharmaceutically acceptable carrier.

The preparations of the present invention may be given orally, parenterally, topically, or rectally. They are of course given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administrations are preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts. A daily, weekly, or monthly dosage (or other time interval) can be used.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required to achieve the desired therapeutic effect and then gradually increasing the dosage until the desired effect is achieved.

If desired, the effective daily dose of the active compound may be administered as two, three, four, five, six or more sub-doses administered separately at appropriate intervals throughout the day, optionally, in unit dosage forms.

While it is possible for a compound of the present invention to be administered alone, it is preferable to administer the compound as a pharmaceutical formulation (composition).

In another aspect, the present invention provides pharmaceutically acceptable compositions which comprise a therapeutically effective amount of one or more of the subject compounds, as described above, formulated together with one or more pharmaceutically acceptable carriers (additives) and/or diluents. As described in detail below, the pharmaceutical compositions of the present invention may be specially formulated for administration in solid or liquid form, including those adapted for the following: oral administration, for example, drenches (aqueous or non-aqueous solutions or suspensions), tablets, boluses, powders, granules, pastes for application to the tongue; parenteral administration, for example, by subcutaneous, intramuscular or intravenous injection as, for example, a sterile solution or suspension; topical application, for example, as a cream, ointment or spray applied to the skin, lungs, or oral cavity; or intravaginally or intravectally, for example, as a pessary, cream or foam; sublingually; ocularly; transdermally; nasally; pulmonary or to other mucosal surfaces.

The compounds according to the invention may be formulated for administration in any convenient way for use in human or veterinary medicine, by analogy with other pharmaceuticals.

Furhter descriptions of suitable pharmaceutically acceptable salts can be found in Handbook of Pharmaceutical Salts, Properties, Selection and Use, Wiley VCH (2002). Furthermore, Remington's Pharmaceutical Sciences, by E. W. Martin, Mack Publishing Co., Easton, PA, 19th Edition (1995), describes compositions and formulations suitable for pharmaceutical delivery of the compounds herein disclosed.

The pharmaceutical compositions can be administered to subjects by a variety of mucosal administration modes, including by oral, rectal, intraocular, intranasal, intrapulmonary, or transdermal delivery, or by topical delivery to other surfaces. Optionally, the compositions can be administered by non-mucosal routes, including by intramuscular, intraocular, subcutaneous, intravenous, intra-arterial, intra-articular, intraperitoneal, intrathecal, intracerebroventricular, or parenteral routes.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of' and "administering a" compound should be understood to mean providing a compound, a prodrug of a compound, or a pharmaceutical composition as described herein. The compound or composition can be administered by another person to the subject (e.g., intravenously) or it can be self-administered by the subject (e.g., tablets).

In the context of the invention, administration of the compound can relate in particular to the administration or provision of the compound to the person donating the organ, for example in order to provide the compound to the organ even before removal from the donor's body, or administration to the organ recipient. Furthermore, administration can refer to direct provision to the organ, either while it is the body of the donor or the recipient, or while it is outside the body after removal from the donor and before transplantation to the recipient. There are several established ways of administering compounds and substances to organs while they are stored outside the body, for example by incubating/storing the organ in a suitable medium or buffer comprising the compound, and/or by perfusing the organ with such a suitable medium or buffer.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 12 or fewer carbon atoms in its backbone (e.g., C₁-C₁₂ for straight chain, C₃-C₁₂ for branched chain), and more preferably 6 or fewer, and even more preferably 4 or fewer. Likewise, preferred cycloalkyls have from 3-10 carbon atoms in their ring structure, and more preferably have 5, 6 or 7 carbons in the ring structure.

Unless the number of carbons is otherwise specified, "lower alkyl" as used herein means an alkyl group, as defined above, but having from one to ten carbons, more preferably from one to six carbon atoms in its backbone structure, and even more preferably from one to four carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

As used herein, the term "halogen" designates -F, -CI, -Br or -I; the term "sulfhydryl" means -SH; and the term "hydroxyl" means -OH.

The term "methyl" refers to the monovalent radical -CH₃, and the term "methoxyl" refers to the monovalent radical -CH₂OH.

The term "aralkyl" or "arylalkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

The term "aryl" as used herein includes 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring can be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls.

The terms ortho, meta and para apply to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" or "heteroaryl" refer to 3- to 10-membered ring structures, more preferably 3- to 7-membered rings, whose ring structures include one to four heteroatoms. Heterocycles can also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, heterocyclyl, aromatic or heteroaromatic moiety, -CF₃, -CN, or the like.

As used herein, the definition of each expression, e.g. alkyl, m, n, etc., when it occurs more than once in any structure, is intended to be independent of its definition elsewhere in the same structure.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents can be one or more and the same or different for appropriate organic compounds, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, heterocyclyl, aromatic or heteroaromatic moiety, -CF₃, -CN, or the like. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

Certain compounds of the present invention may exist in particular geometric or stereoisomeric forms. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention. In certain embodiments, the present invention relates to a compound represented by any of the structures outlined herein, wherein the compound is a single stereoisomer.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Contemplated equivalents of the compounds described above include compounds which otherwise correspond thereto, and which have the same general properties thereof (e.g., functioning as inhibitors of cellular necrosis), wherein one or more simple variations of substituents are made which do not adversely affect the efficacy of the compound. In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants, which are in themselves known, but are not mentioned here.

The term "alkoxy" refers to a straight, branched or cyclic hydrocarbon configuration and combinations thereof, including from 1 to 20 carbon atoms, preferably from 1 to 8 carbon atoms, more preferably from 1 to 4 carbon atoms, that include an oxygen atom at the point of attachment (such as O-alkyl). An example of an "alkoxy group" is represented by the formula -OR, where R can be an alkyl group, optionally substituted with an alkenyl, alkynyl, aryl, aralkyl, cycloalkyl, halogenated alkyl, or heterocycloalkyl group. Suitable alkoxy groups include methoxy, ethoxy, n-propoxy, i-propoxy, n-butoxy, i-butoxy, sec-butoxy, tert-butoxy cclopropoxy, cyclohexyloxy, and the like.

"Carbonyl" refers to a radical of the formula -C(O)-. Carbonyl-containing groups include any substituent containing a carbon-oxygen double bond (C=O), including acyl groups, amides, carboxy groups, esters, ureas, carbamates, carbonates and ketones and aldehydes, such as substituents based on -COR or -RCHO where R is an aliphatic, heteroaliphatic, alkyl, heteroalkyl, hydroxyl, or a secondary, tertiary, or quaternary amine.

"Alkoxycarbonyl" refers to an alkoxy substituted carbonyl radical (such as -C(O)OR), wherein R represents an optionally substituted alkyl, aryl, aralkyl, cycloalkyl, cycloalkylalkyl or similar moiety.

The term "alkyl amino" refers to alkyl groups as defined above where at least one hydrogen atom is replaced with an amino group.

"Aminocarbonyl" alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like. An aminocarbonyl group may be -N(R)-C(O)-R (wherein R is a substituted group or H) or -C(O)-N(R).

"Carboxyl" refers to a -COOH radical. Substituted carboxyl refers to -COOR where R is aliphatic, heteroaliphatic, alkyl, heteroalkyl, or a carboxylic acid or ester.

The term "hydroxyl" is represented by the formula -OH. The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

The term "amine" refers to a group of the formula -NRR', where R and R' can be, independently, hydrogen or an alkyl, alkenyl, alkynyl, aryl, aralkyl, cycloalkyl, halogenated alkyl, or heterocycloalkyl group described above.

The term "amide" or "amido" is represented by the formula -C(O)NRR', where R and R' independently can be a hydrogen, alkyl, alkenyl, alkynyl, aryl, aralkyl, cycloalkyl, halogenated alkyl, or heterocycloalkyl group described above. A suitable amido group is acetamido.

The term "aralkyl" refers to an aryl group having an alkyl group, as defined above, attached to the aryl group, as defined above. An example of an aralkyl group is a benzyl group.

Optionally substituted groups, such as "optionally substituted alkyl," refers to groups, such as an alkyl group, that when substituted, have from 1-5 substituents, typically 1, 2 or 3 substituents, selected from alkoxy, optionally substituted alkoxy, acyl, acylamino, acyloxy, amino, aminoacyl, aminoacyloxy, aryl, carboxyalkyl, optionally substituted cycloalkyl, optionally substituted cycloalkenyl, halogen, optionally substituted heteroaryl, optionally substituted heterocyclyl, hydroxy, sulfonyl, thiol and thioalkoxy. In particular, optionally substituted alkyl groups include, by way of example, haloalkyl groups, such as fluoroalkyl groups, including, without limitation, trifluoromethyl groups. These potential optional substituents apply to any group of the formula disclosed herein where an optional substituent is recited. Preferable optional substituents are hydroxyl, alkyl, alkoxy, carbonyl, alkoxycarbonyl, NO₂, amine.

For purposes of this invention, the chemical elements are identified in accordance with the Periodic Table of the Elements, CAS version, Handbook of Chemistry and Physics, 67th Ed., 1986-87, inside cover.

Protected derivatives of the disclosed compound also are contemplated. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

It is understood that substituents and substitution patterns of the compounds described herein can be selected by one of ordinary skill in the art to provide compounds that are chemically stable and that can be readily synthesized by techniques known in the art and further by the methods set forth in this disclosure.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention but represent preferred embodiments of aspects of the invention provided for greater illustration of the invention described herein.

### Brief description of the figures:

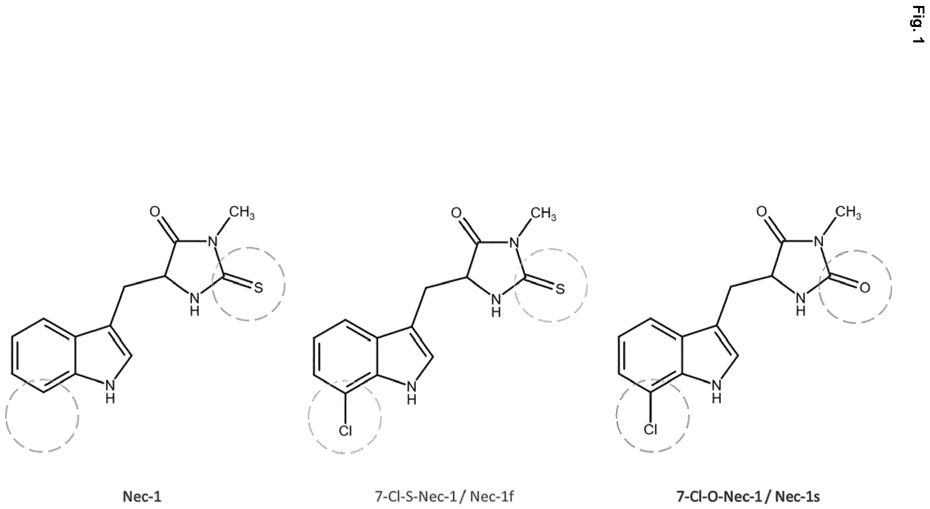

**Figure 1****:** *Overview of chemical structures of different necrostatins.* Structural differences between Nec-1f in comparison to known necroptosis inhibitors Nec-1 and Nec-1s. Highlighted are the main residues involved in facilitating protection from necroptosis and/or ferroptosis.

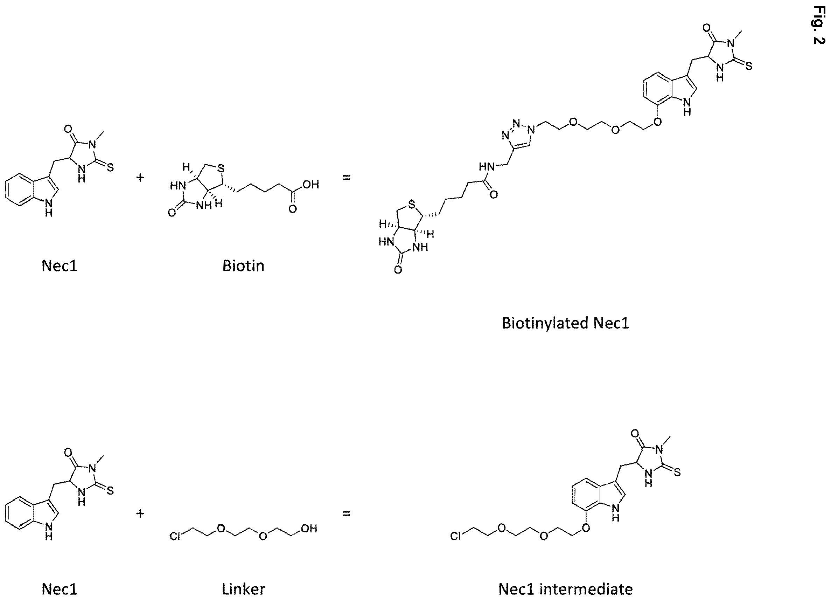

**Figure 2****:** Biotinylation of Nec-1. Simplified scheme of linking the target molecule Nec-1 to a biotin anker. Nec-1 is biotinylated by adding a spacer to position 7 of the bicyclic indole structure and linking the biotin via a click reaction to the spacer.

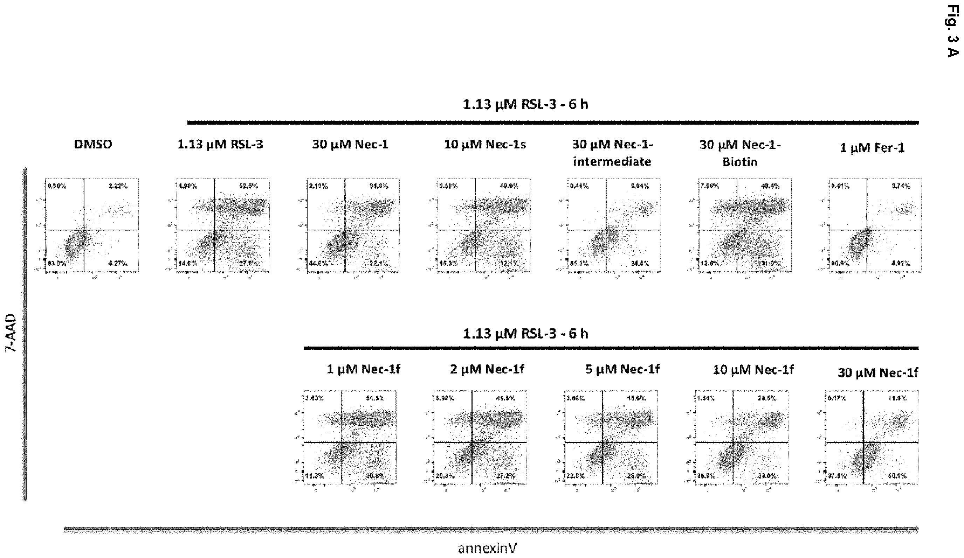

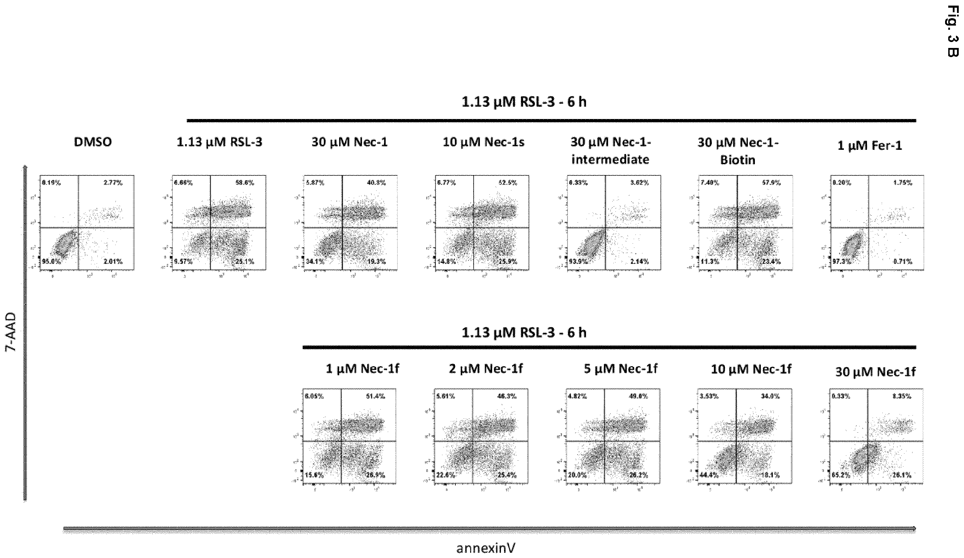

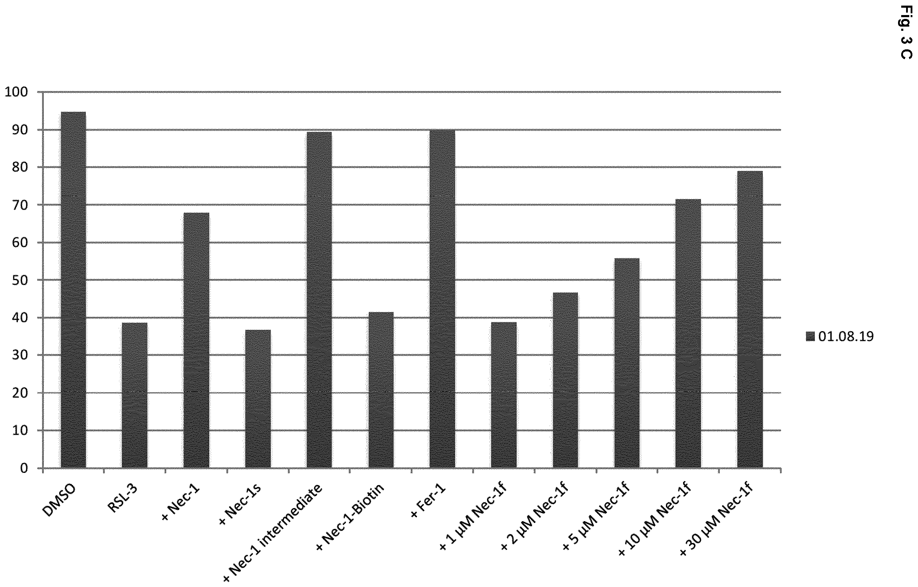

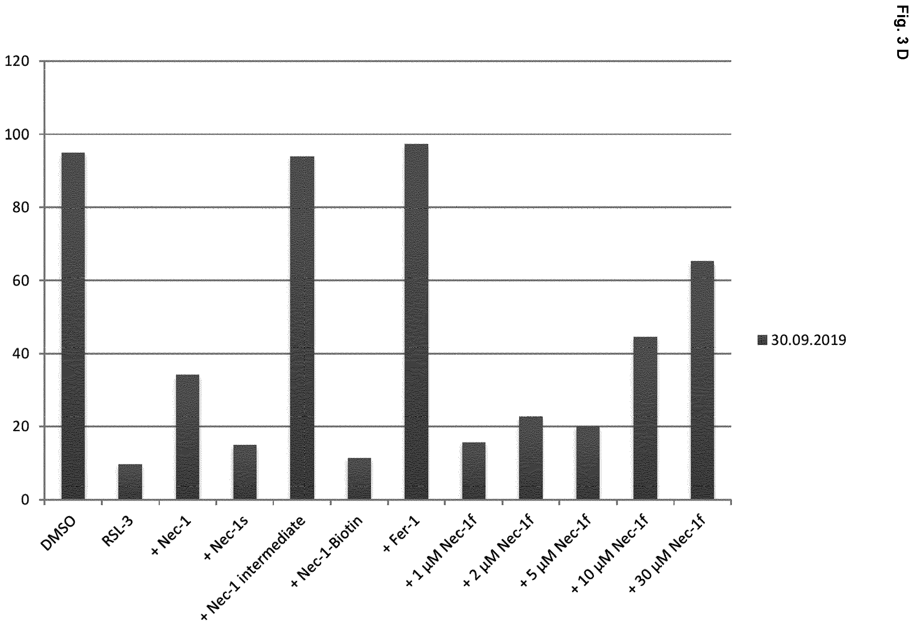

**Figure 3****:** Nec-1f protects cells from undergoing ferroptosis. (A, B) Inhibition of cell death by Nec-1f in NIH3T3 cells was assessed by flow cytometry. Cells were treated with the ferroptosis inducer RSL-3 (1.13 µM) for 6 h with or without simultaneous substitution of necrostatins. The percentage of cells positive for annexinV and 7-AAD was recorded. (C, D) Viability of NIH3T3 cells after induction of ferroptosis. Cells were treated with 1.13 µM RSL-3 for 6 h and collected for flow cytometry. Cell viability was assessed by staining for 7-AAD and annexinV and the percentage of double-negative events were plotted in this graph.

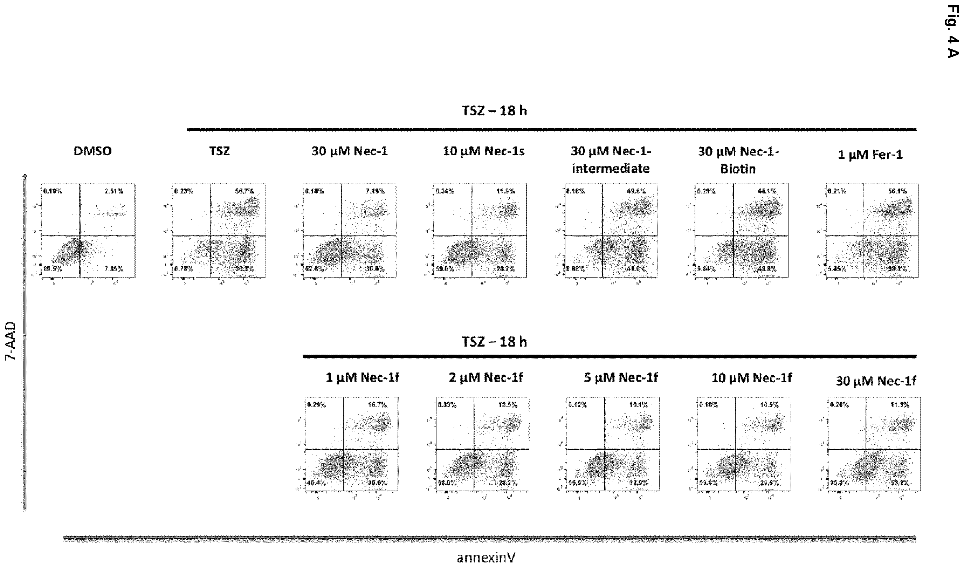

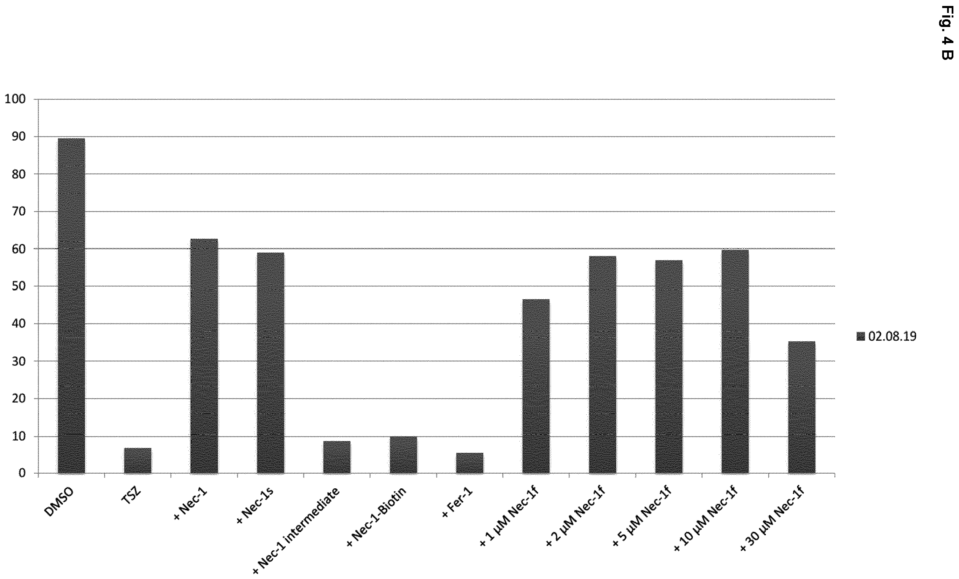

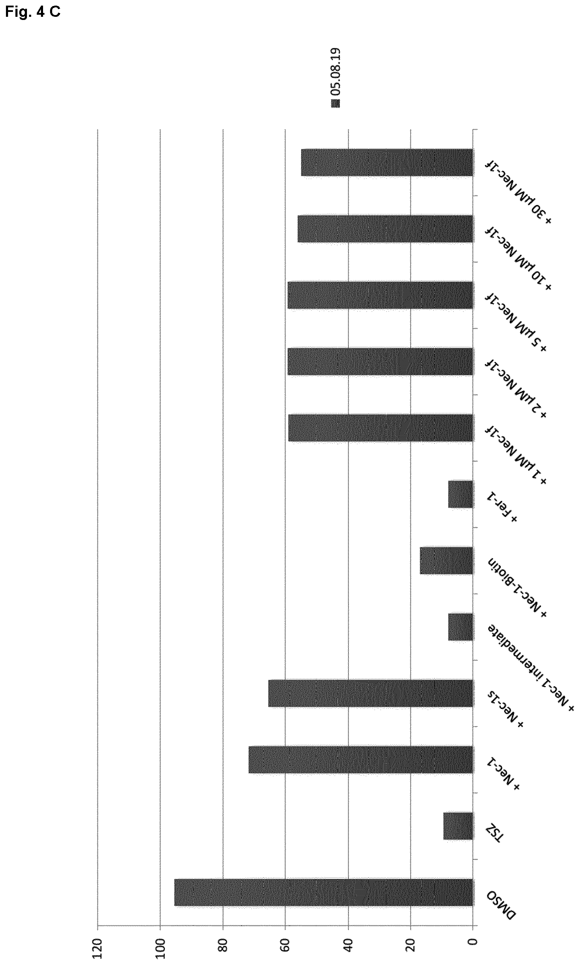

**Figure 4****:** Nec-1f protects cells from undergoing necroptosis. (A, B) Inhibition of necroptotic cell death by Nec-1f in NIH3T3 cells was assessed by flow cytometry. Cells were treated with the TNF, Birinapant and z-VAD-fmk (TSZ) to induce necroptosis. After 18 h necroptosis induction with or without simultaneous substitution of necrostatins the cells were harvested and stained for flow cytometry. The percentage of cells positive for annexinV and 7-AAD was recorded. (C, D) Viability of NIH3T3 cells 18h after induction of necroptosis via TSZ treatment. Cells viability is displayed as percentage in the double-negative fraction after staining for 7-AAD and annexinV.

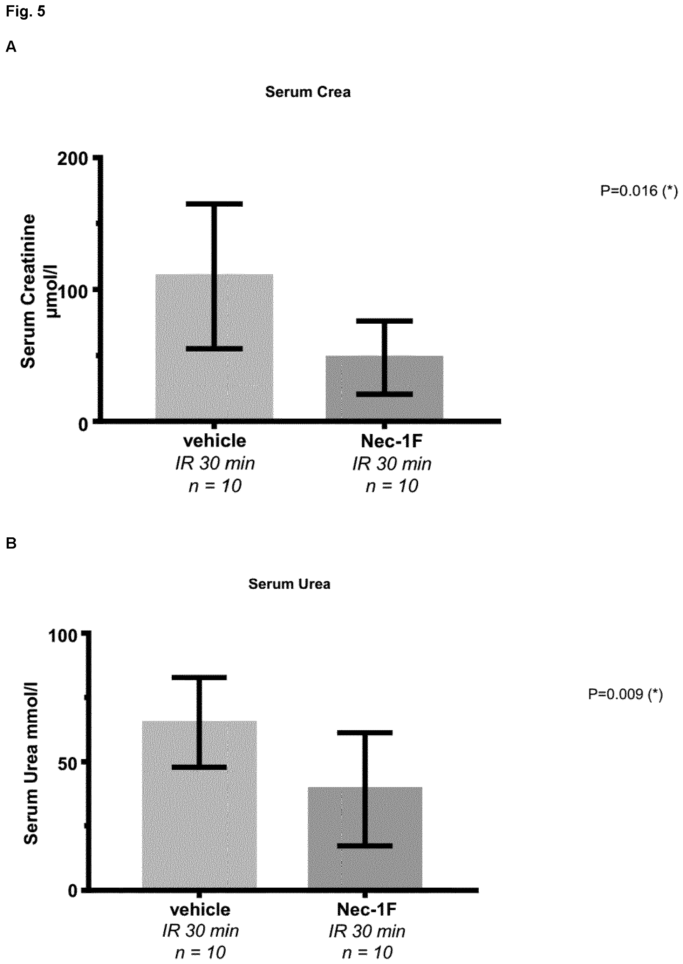

**Figure 5****:** Nec-1f is protective in an animal model of renal ischemia-reperfusion injury. Animals were subjected to 30 min of ischemia after i.p. injection of 0.6 mg Nec-1f per kg bodyweight or the respective vehicle control in PBS. (A, B) After 48h mice were sacrificed and serum creatinine levels and serum urea levels were measured as markers of renal function and intoxication. Error bars represent standard deviation.
**Figure 6****:** Synthesis of 5-[(7-Chloro-1H-indol-3-yl)methyl]-3-methyl-2-thioxo-imidazolidin-4-one (1-3; Nec-1f) according to example 2.
**Figure 7****:** Synthesis of 5-[(3aS,4R,6aR)-2-Oxo-1,3,3a,4,6,6a-hexahydrothieno[3,4-d]imidazol-4-yl]-N-[[1-[2-[2-[2-[[3-[(1-methyl-5-oxo-2-thioxo-imidazolidin-4-yl)methyl]-1H-indol-7-yl]oxy]ethoxy]ethoxy]ethyl]triazol-4-yl]methyl]pentanamide (2-8; biotinylated Nec-1) according to example 3.

### EXAMPLES

The invention is further described by the following examples. These are not intended to limit the scope of the invention but represent preferred embodiments of aspects of the invention provided for greater illustration of the invention described herein.

### Example 1: Inhibition of necroptosis and ferroptosis

### Methods employed in the Examples of inhibiting necroptosis and ferroptosis

### Reagents, cell lines and cell death assays

Necrostatin (Nec-1) was obtained from Sigma-Aldrich. The zVAD-fmk (herein referred to as zVAD) was purchased from BD Biosciences. SMAC-mimetics (Birinapant) was from Absource Diagnostics (Selleckchem). Murine NIH3T3 fibroblasts were originally obtained from ATCC and were cultured in Dulbecco's modified Eagle medium (DMEM) (Invitrogen) supplemented with 10% (vol/vol) FCS, 100 U/mL penicillin, and 100 µg/mL streptomycin. Cell lines were cultured in a humidified 5% CO2 atmosphere. For induction of necroptosis, NIH3T3 cells were stimulated for 24 h at 37 °C with 100 ng/mL TNFα plus 1 µM SMAC-mimetics (Birinapant) plus 25 µM zVAD as indicated (vehicle-treated cells served as control). Biological and chemical reagents were from the following sources: Necrostatin 1 (BioVision), zVAD.fmk (Bachem).

### Fluorescence-Activated Cell Sorting.

Phosphatidylserine exposure to the outer cell membrane of apoptotic cells or at the inner plasma membrane of necrotic cells and incorporation of 7-AAD into necrotic cells were quantified by fluorescence-activated cell sorting (FACS) analysis. The ApoAlert annexin V-FITC antibody and the 7-AAD antibody were purchased from BD Biosciences.

### Mice

All WT mice reported in this study were on C57BL/6 background. Eight to 12-wk-old male C57BL/6 mice (average weight ∼23 g) were used for all WT experiments, unless otherwise specified. All *in vivo* experiments were performed according to the Protection of Animals Act. In all experiments, mice were rigorously matched for age, sex and weight.

### Murine ischemia-reoerfusion injury

Induction of kidney IRI was performed via a midline abdominal incision and a bilateral renal pedicle clamping for 30 min (mild IRI) using microaneurysm clamps (Aesculab). Throughout the surgical procedure, the body temperature was maintained between 36 °C and 37 °C by continuous monitoring using a temperature-controlled self-regulated heating system (Fine Science Tools). After removal of the clamps, reperfusion of the kidneys was confirmed visually. The abdomen was closed in two layers using standard 6-0 sutures. Sham-operated mice underwent the identical surgical procedures, except that microaneurysm clamps were not applied. To maintain fluid balance, all of the mice were supplemented with 1 mL of prewarmed PBS administered intraperitoneally directly after surgery. All mice were killed 48 h after reperfusion for each experiment. All ischemia-reperfusion experiments were performed in a double-blinded manner. Where indicated, 0.6 mg Nec-1f per kg bodyweight was applied intraperitoneally 15 min before the onset of ischemia in a final volume of 200 µl. In those experiments, control mice received 200µl of PBS supplemented with respective amount of DMSO.

### Statistics

For all experiments, differences of datasets were considered statistically significant when P values were lower than 0.05, if not otherwise specified. Statistical comparisons were performed using the two-tailed Student t test. Asterisks are used in the figures to specify statistical significance (*P < 0.05; **P < 0.02; ***P < 0.001). Statistics are indicated as SD unless otherwise specified.

### Results of the Examples of inhibiting necroptosis and ferroptosis

### Inhibition of ferroptosis

In all experiments tested, in cell culture all assays used to detect ferroptosis were reversed by the compound Nec-1f.

### Inhibition of necroptosis

In all experiments tested, in cell culture all assays used to detect necroptosis were reversed by the compound Nec-1f.

### Nec-1f is protective in ischemia-reperfusion injury in mice

Kidney ischemia-reperfusion injury (kidney-IRI) results from the combined activation of necroptosis and ferroptosis pathways (see above). Nec-1f reversed all measured markers of kidney-IRI including intoxication markers (serum urea levels) and markers of renal function (serum creatinine levels).

### Conclusions of the Examples of inhibiting necroptosis and ferroptosis

We conclude that Nec-1f is a potent inhibitor of both ferroptosis and necroptosis. It may represent an ideal compound for the treatment and/or prevention of acute organ injury and other diseases.

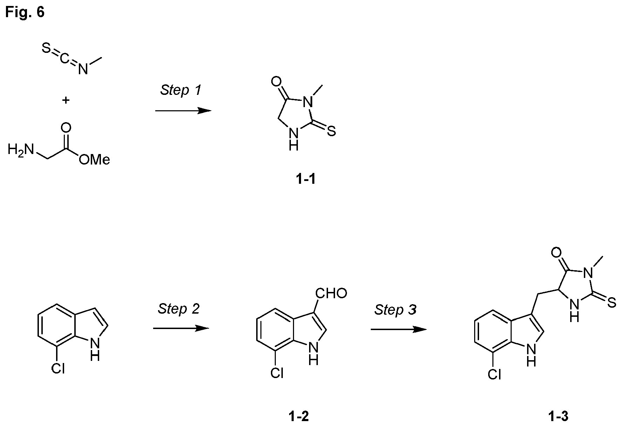

### Example 2: 5-[(7-Chloro-1H-indol-3-yl)methyl]-3-methyl-2-thioxo-imidazolidin-4-one (1-3; Nec-1f) (as depicted in Figure 6)

### Step 1. 3-Methyl-2-thioxo-imidazolidin-4-one (1-1)

H-Gly-OMe x HCI (6.28 g, 50 mmol), methyl isothiocyanate (3.66 g, 3.42 mL, 50 mmol), and Et₃N (5.06 g, 6.93 mL, 50 mmol) was stirred in dry Et₂O (100 mL) at rt under an argon atmosphere for 72 h. Then, volatiles were removed and EtOAc (300 mL) was added. The suspension was filtered and the filtrate was dried over Na₂SO₄. After filtration and evaporation of the solvent, the crude material was purified by column chromatography (petroleum ether/EtOAc 1:2) to give 3-methyl-2-thioxo-imidazolidin-4-one (**1-1**) (3.10 g, 70%) as an amber solid. ¹H NMR (600 MHz, DMSO-*d₆*) δ 3.03 (s, 3H), 4.10 (s, 2H), 10.12 (br s, 1H); ¹³C NMR (151 MHz, DMSO-*d₆*) δ 26.90, 48.69, 172.85, 183.89; MS *m*/*z*: 131.2 [M + H]⁺.

### Step 2. 7-Chloro-1H-indole-3-carbaldehyde (1-2)

Dry DMF (10 mL) was cooled to 0 °C and POCl₃ (1.63 g, 1.0 mL, 10.6 mmol) was dropwise added under an argon atmosphere. After stirring for 30 min at this temperature, a solution of 7-chloroindole (1.52 g, 10 mmol) in dry DMF (10 mL) was added and the combined mixture was stirred at rt for 3 h. Subsequently, half-saturated NaHCO₃ solution (200 mL) was added and the mixture was vigorously stirred together with EtOAc (200 mL) for 10 min. The, 2N NaOH solution (100 mL) was added and the mixture was heated to 80-90 °C for 10 min. After cooling, the organic layer was separated and the aqueous solution was extracted again with EtOAc (200 mL). The combined organic layers were dried over Na₂SO₄, filtered, and concentrated *in vacuo.* The crude material was purified by column chromatography (gradient of petroleum ether/EtOAc 2:1 to 1:1) to give 7-chloro-1*H*-indole-3-carbaldehyde (**1-2**) (1.29 g, 72%) as an amber solid. ¹H NMR (600 MHz, DMSO-*d₆*) δ 7.22 (t, *J* = 7.8 Hz, 1H), 7.34 (dd, *J* = 0.9, 7.7 Hz, 1H), 8.05 (dd, *J* = 0.9, 7.9 Hz, 1H), 8.36 (s, 1H), 9.96 (s, 1H), 12.50 (s, 1H); ¹³C NMR (151 MHz, DMSO-*d₆*) δ 116.81, 119.03, 119.93, 123.22, 123.47, 126.17, 134.08, 139.27, 185.48; MS *m*/*z:* 179.8 [M + H]⁺.

### Step 3. 5-[(7-Chloro-1H-indol-3-yl)methyl]-3-methyl-2-thioxo-imidazolidin-4-one (1-3; Nec-1f)

*N*-methylmorpholine (10 mL) was degased in an ultrasonic bath, followed by purging with argon. Subsequently, 3-methyl-2-thioxo-imidazolidin-4-one **(1-1)** (1.63 g, 12.5 mmol), 7-chloro-1*H-*indole-3-carbaldehyde (**1-2**) (0.90 g, 5 mmol), and dithiothreitol (0.77 g, 5 mmol) were added. The mixture was stirred at 110 °C under an argon atmosphere for 4 h. After cooling, the mixture was diluted with 1N HCl (100 mL) and it was extracted with EtOAc (3 × 75 mL). The cominbed organic layers were washed with brine (100 mL), dried over Na₂SO₄, filtered, and concentracted *in vacuo.* The crude material was purified by column chromatography (gradient of petroleum ether/EtOAc 4:1 to 1:1) and product enriched fractions were pooled and subjected to additional purification via HPLC (40% to 90% v/v acetonitrile) to give Nec-1f **(1-3)** (69 mg, 5%) as a yellow solid after lyophilisation. Mp 248 - 252 °C; ¹H NMR (600 MHz, DMSO-de) δ 2.85 (s, 3H), 3.15 (d, *J* = 5.2 Hz, 2H), 4.57 (t, *J* = 5.1 Hz, 1H), 6.98 (t, *J* = 7.7 Hz, 1H), 7.13 (d, *J* = 7.5 Hz, 1H), 7.16 (d, *J* = 2.5 Hz, 1H), 7.50 (d, *J* = 7.9 Hz, 1H), 10.32 (br s, 1H), 11.26 (br s, 1H); ¹³C NMR (151 MHz, DMSO-*d₆*) δ 26.12, 26.79, 59.64, 109.04, 115.84, 117.75, 119.64, 120.66, 125.63, 129.39, 132.80, 174.61, 183.04; MS *m*/*z:* 294.0 [M + H]⁺.

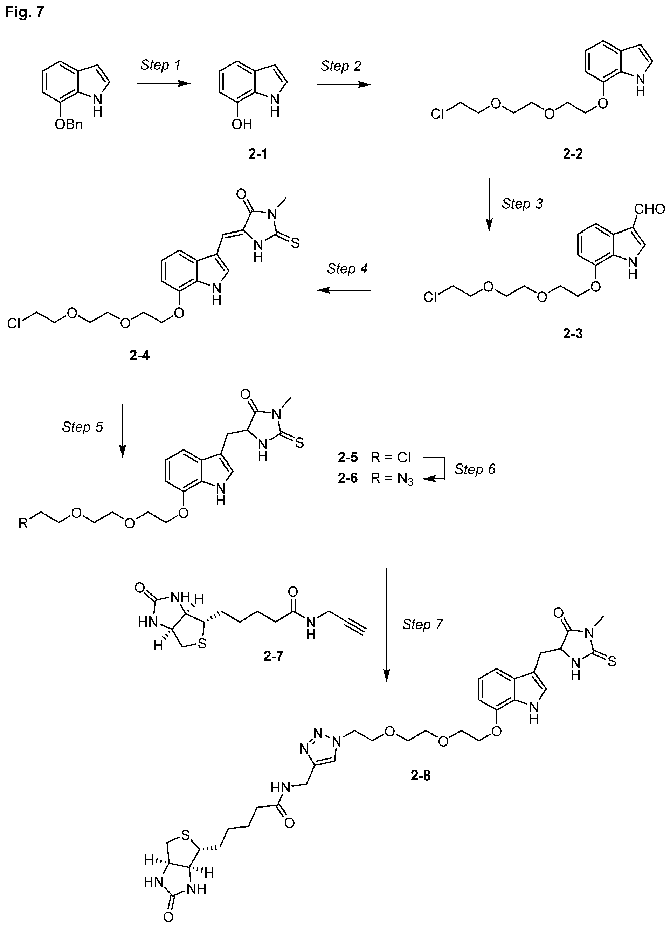

### Example 3: 5-[(3aS,4R,6aR)-2-Oxo-1,3,3a,4,6,6a-hexahydrothieno[3,4-d]imidazol-4-yl]-N-[[1-[2-[2-[2-[[3-[(1-methyl-5-oxo-2-thioxo-imidazolidin-4-yl)methyl]-1H-indol-7-yl]oxy]ethoxy]ethoxy]ethyl]triazol-4-yl]methyl]pentanamide (2-8; biotinylated Nec-1) (as depicted in Figure 7)

### Step 1. 1H-indol-7-ol (2-1)

7-Benzyloxyindole (2.24 g, 10 mmol) was hydrogenated over 10% palladium on carbon (0.22 g) in dry EtOAc (50 mL) at ambient temperature under atmospheric pressure overnight to give 1*H-*indol-7-ol **(2-1)** as a tan solid in quantitative yield after filtration and evaporation of the solvent. ¹H NMR (500 MHz, DMSO-*d₆*) δ 6.33 (dd, *J* = 2.0, 3.0 Hz, 1H), 6.47 (dd, *J* = 0.9, 7.5 Hz, 1H), 6.71 - 6.79 (m, 1H), 6.94 - 7.00 (m, 1H), 7.18 (t, *J* = 2.7 Hz, 1H), 9.41 (s, 1H), 10.81 (br s, 1H); ¹³C NMR (126 MHz, DMSO-*d₆*) δ 101.42, 105.32, 111.24, 119.52, 124.63, 126.16, 129.71, 143.69; *MS m*/*z:* 134.0 [M + H]⁺.

### Step 2. 7-[2-[2-(2-Chloroethoxy)ethoxy]ethoxy]-1H-indole (2-2)

To a solution of 2-[2-(2-chloroethoxy)ethoxy]ethanol (1.85 g, 11 mmol) and 1*H*-indol-7-ol **(2-1)** (1.33 g, 10 mmol) in dry and degassed THF (100 mL) was added triphenylphosphine (2.89 g, 11 mmol). Subsequently, the vessel was lowered into an ultrasonic bath and while sonicating, diisopropyl azodicarboxylate (2.22 g, 2.18 mL, 11 mmol) was added dropwise. The mixture was sonicated for 30 min and then the volatiles were removed in vacuo. The crude material was purified by column chromatography (gradient of petroleum ether/EtOAc 10:1 to 2:1) to give 7-[2-[2-(2-chloroethoxy)ethoxy]ethoxy]-1*H*-indole (2-2) (2.47 g, 87%) as an amber oil. MS *m*/*z:* 283.9 [M + H]⁺.

### Step 3. 7-[2-[2-(2-Chloroethoxy)ethoxy]ethoxy]-1H-indole-3-carbaldehyde (2-3)

Phosphoroxychlorid (1.63 g, 1.0 mL, 10.6 mmol) was stirred in dry DMF (10 mL) at 0°C under an argon atmosphere for 30 min. Then, 7-[2-[2-(2-chloroethoxy)ethoxy]ethoxy]-1*H*-indole **(2-2)** (2.27 g, 8 mmol) in dry DMF (8 mL) was added and it was stirred at rt for 3 h. The mixture was poured into a mixture of half-saturated NaHCO₃ solution (80 mL) and EtOAc (80 mL) while vigorously stirring. Subsequently, 2N NaOH (8 mL) was added and it was gently heated until the mixture became clear. It was transferred into a separation funnel, and the aqueous phase was extracted once again with EtOAc (80 mL). The combined organic layers were washed with H₂O (80 mL) and brine (80 mL), and it was dried over Na₂SO₄, filtered and evaporated. The crude material was purified by column chromatography (gradient of petroleum eter/EtOAc 1:2 to EtOAc) to give 7-[2-[2-(2-chloroethoxy)ethoxy]ethoxy]-1H-indole-3-carbaldehyde **(2-3)** (1.65 g, 66%) as a colorless solid. Mp 46-48 °C; ¹H NMR (600 MHz, DMSO-*d₆*) δ 3.56 - 3.61 (m, 2H), 3.62 - 3.69 (m, 6H), 3.82 - 3.87 (m, 2H), 4.26 - 4.31 (m, 2H), 6.84 (d, *J* = 7.8 Hz, 1H), 7.11 (t, *J* = 7.9 Hz, 1H), 7.65 (d, *J* = 7.9 Hz, 1H), 8.15 (s, 1H), 9.92 (s, 1H), 12.20 (br s, 1H); ¹³C NMR (151 MHz, DMSO-*d₆*) δ 43.70, 67.91, 69.15, 69.90, 70.12, 70.71, 105.45, 113.60, 118.82, 123.11, 127.27, 137.83, 145.57, 185.24; MS *m*/*z:* 312.0 [M + H]⁺.

### Step 4. (5Z)-5-[[7-[2-[2-(2-Chloroethoxy)ethoxy]ethoxy]-1H-indol-3-yl]methylene]-3-methyl-2-thioxo-imidazolidin-4-one (2-4)

*N*-methylmorpholine (10 mL) was degased in an ultrasonic bath, followed by purging with argon. Subsequently, 3-methyl-2-thioxo-imidazolidin-4-one **(1-1)** (1.63 g, 12.5 mmol), and 7-[2-[2-(2-chloroethoxy)ethoxy]ethoxy]-1H-indole-3-carbaldehyde **(2-3)** (1.60 g, 5 mmol) were added. The mixture was stirred at 110 °C under an argon atmosphere for 4 h. After cooling, the mixture was diluted with 1N HCl (100 mL) and it was extracted with EtOAc (3 x 75 mL). The cominbed organic layers were washed with brine (100 mL), dried over Na₂SO₄, filtered, and concentracted *in vacuo.* The crude material was purified by column chromatography (gradient of petroleum ether/EtOAc 2:1 to 1:1) to give (5*Z*)-5-[[7-[2-[2-(2-chloroethoxy)ethoxy]ethoxy]-1*H*-indol-3-yl]methylene]-3-methyl-2-thioxo-imidazolidin-4-one (**2-4**) (0.68 g, 32%) as an orange solid. MS *m*/*z:* 424.1 [M + H]⁺.

### Step 5. 5-[[7-[2-[2-(2-Chloroethoxy)ethoxy]ethoxy]-1H-indol-3-yl]methyl]-3-methyl-2-thioxo-imidazolidin-4-one (2-5, Nec-1 intermediate)

SiO₂ (1.5 g) was activated by heating it at 120 °C for 5 h. Subsequently, it was added to a mixture of (5*Z*)-5-[[7-[2-[2-(2-chloroethoxy)ethoxy]ethoxy]-1*H*-indol-3-yl]methylene]-3-methyl-2-thioxo-imidazolidin-4-one **(2-4)** (0.63 g, 1.5 mmol) and diethyl 1,4-dihydro-2,6-dimethyl-3,5-pyridinedicarboxylate (0.49 g, 1.95 mmol) in dry toluene (45 mL). The mixture was heated under an argon atmosphere for 24 h at 100 °C. After cooling, the solvent was evaporated and the crude material was purified by column chromatography (gradient of petroleum ether/EtOAc 2:1 to 1:1) to give 5-[[7-[2-[2-(2-chloroethoxy)ethoxy]ethoxy]-1H-indol-3-yl]methyl]-3-methyl-2-thioxo-imidazolidin-4-one **(2-5, Nec-1 intermediate)** (0.42 g, 66%) as an orange oil. ¹H NMR (500 MHz, DMSO-*d₆*) δ 2.85 (s, 3H), 3.12 (d, *J* = 5.0 Hz, 2H), 3.57 - 3.71 (m, 8H), 3.77 - 3.87 (m, 2H), 4.15 - 4.24 (m, 2H), 4.50 - 4.55 (m, 1H), 6.61 (d, *J* = 7.6 Hz, 1H), 6.86 (t, *J* = 7.8 Hz, 1H), 6.98 (d, *J* = 2.5 Hz, 1H), 7.11 (d, *J* = 8.0 Hz, 1H), 10.28 (d, *J* = 1.3 Hz, 1H), 10.85 (d, *J* = 2.8 Hz, 1H); ¹³C NMR (126 MHz, DMSO-*d₆*) δ 26.24, 26.79, 43.72, 59.77, 67.61, 69.27, 69.90, 70.13, 70.72, 102.77, 107.97, 111.56, 119.08, 123.94, 126.20, 129.13, 145.22, 174.69, 183.00; MS *m*/*z*: 426.2 [M + H]⁺.

### Step 6. 5-[[7-[2-[2-(2-Azidoethoxy)ethoxy]ethoxy]-1H-indol-3-yl]methyl]-3-methyl-2-thioxo-imidazolidin-4-one (2-6)

Nec-1 intermediate **(2-5)** (0.42 g, 1.0 mmol) in dry DMF (20 mL) was purged with argon for 30 min. Subsequently, dithiothreitol (0.15 g, 1.0 mmol) and sodium azide (0.52 g, 8 mmol) were added. The mixture was heated to 60 °C for 24 h in a brown round bottom flask. After cooling, the mixture was diluted with EtOAc (150 mL) and half-saturated brine (150 mL). The organic layer was separated and the aqueous layer was extracted with further portions of EtOAc (2 x 50 mL). The combined organic layers were washed with brine (100 mL), dried over Na₂SO₄, filtered and evaporated. The crude material was purified by column chromatography (gradient of petroleum ether/EtOAc 2:1 to 1:1) to give 5-[[7-[2-[2-(2-azidoethoxy)ethoxy]ethoxy]-1H-indol-3-yl]methyl]-3-methyl-2-thioxo-imidazolidin-4-one **(2-6)** (143 mg, 33%) as an orange oil. MS *m*/*z:* 433.0 [M + H]⁺.

### Step 7. 5-[(3aS,4R,6aR)-2-Oxo-1,3,3a,4,6,6a-hexahydrothieno[3,4-d]imidazol-4-yl]-N-[[1-[2-[2-[2-[[3-[(1-methyl-5-oxo-2-thioxo-imidazolidin-4-yl)methyl]-1H-indol-7-yl]oxy]ethoxy]ethoxy]ethyl]triazol-4-yl]methyl]pentanamide (2-8; biotinylated Nec-1)

5-[[7-[2-[2-(2-Azidoethoxy)ethoxy]ethoxy]-1H-indol-3-yl]methyl]-3-methyl-2-thioxo-imidazolidin-4-one **(2-6)** (108 mg, 0.25 mmol) and 5-[(3a*S*,4*R*,6a*R*)-2-oxo-1,3,3a,4,6,6a-hexahydrothieno[3,4-d]imidazol-4-yl]-*N*-prop-2-ynyl-pentanamide (2-7) (70 mg, 0.25 mmol) were dissolved in dry, degassed DMSO (5 mL each). After combining both mixtures and purging with argon, solutions of sodium ascorbate (50 mL, 0.25 mmol) and CuSO₄ × 5 H₂O (31 mg, 0.125 mmol) in H₂O (1 mL each) were added. The combined mixture was stirred in a brown glass vial for 18 h at rt. The brown solution was quenched by the addition of 10% KHSO₄ solution (100 mL) and it was extracted with CHCl3 (3 × 50 mL). The combined organic layers were washed with 5% LiCI solution and brine (each 50 mL), dried over Na₂SO₄, filtered, and concentracted *in vacuo.* The crude material was purified by HPLC (20% to 80% v/v acetonitrile) to give biotinylated Nec-1 **(2-8)** (9 mg, 5%) as a yellow solid after lyophilisation. ¹H NMR (600 MHz, Chloroform-d) δ 1.09 - 1.76 (m, 6H), 1.99 (d, *J* = 6.0 Hz, 7H), 2.70 (dd, *J* = 9.5, 12.9 Hz, 1H), 2.79 - 2.91 (m, 1H), 2.97 - 3.20 (m, 4H), 3.33 - 3.52 (m, 1H), 3.65 (s, 3H), 3.72 - 3.97 (m, 3H), 4.12 - 4.60 (m, 7H), 5.42 (d, *J* = 31.2 Hz, 2H), 6.64 (dd, *J* = 4.8, 7.7 Hz, 1H), 6.92 - 7.14 (m, 2H), 7.21 (dd, *J* = 3.7, 8.0 Hz, 1H), 7.27 - 7.62 (m, 1H), 7.72 - 7.96 (m, 1H), 9.50 (s, 1H), 9.59 (d, *J* = 14.0 Hz, 1H); HRMS *m*/*z:* 714.2858 [M + H]⁺.

## Claims

1. A compound according to Formula I for use as a medicament in the prevention of organ transplant rejection and/or transplant organ damage, 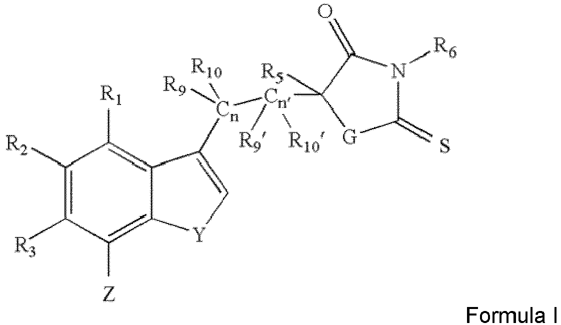 or a stereoisomeric form thereof, a pharmaceutically acceptable acid or base addition salt thereof; wherein
- Y is S or NR₈;
- G is O or NR₇, preferably NH;
- Z is Cl or OR₁₁;
- R₁, R₂, and R₃ are, independently of each other, H, OH, OR₈, F, CI, Br, I, N(R₈)₂, COOH, CO₂R₈, NO₂, NHC(O)R₈, lower alkyl, substituted lower alkyl, aryl, substituted aryl, heteroaryl, or substituted heteroaryl, preferably H;
- R₅, and R₇ are, independently of each other, H or lower alkyl, preferably H;
- R₈ is H, lower alkyl, substituted lower alkyl, aryl, substituted aryl, arylalkyl, alkenyl, alkynyl, heteroaryl, or substituted heteroaryl, preferably H;
- Rg, R₁₀, R₉', R₁₀', are, independently of each other, H, F, Cl, Br, I, lower alkyl, substituted lower alkyl, or a three to six membered cycloalkyl or substituted cycloalkyl that includes Cₙ and/or Cₙ', preferably H;
- R₁₁ is alkyl, polyethylene or arylalkyl or a chemically divers linker structure with a terminal biotin moiety or any other terminal moiety able to bind to a functional protein; and
- n and n' are, independently of each other, an integer from zero to five, wherein the sum of n and n' is preferably one.

2. The compound for use as a medicament according to the preceding claim, wherein the compound inhibits and/or reduces ferroptosis and necroptosis of cells of the transplanted organ.

3. The compound for use as a medicament according to any one of the preceding claims, wherein the compound is administered to the patient and/or the organ within the first 10 days after, preferably 3 days after, more preferably at the time point of or before organ transplantation, such as at the time point of organ removal from the donor.

4. The compound for use as a medicament according to any of the preceding claims, wherein the patient is at risk of transplant rejection, preferably acute transplant rejection.

5. The compound for use as a medicament according to any of the preceding claims, wherein the patient is at risk or shows signs of acute transplant dysfunction.

6. The compound for use as a medicament according to any of the preceding claims, wherein the patient is at risk of or shows signs of ischemia-reperfusion injury and/or necroinflammation.

7. The compound for use as a medicament according to any of the preceding claims, wherein the treatment is administered in advance of organ transplant in prophylactic treatment for the prevention of organ rejection.

8. The compound for use as a medicament according to any of the preceding claims, wherein the compound is administered in combination with immunosuppression.

9. The compound for use as a medicament according to any of the preceding claims, wherein the patient is a kidney transplant patient.

10. The compound for use as a medicament according to any of the preceding claims, wherein the compound inhibits the kinase activity of Receptor-interacting serine/threonine-protein kinase 1 (RIPK1) and lipid peroxidation.

11. The compound for use as a medicament according to any of the preceding claims, wherein the compound inhibits and/or reduces exposure of intracellular antigens of the transplanted organ.

12. The compound for use as a medicament according to any of the preceding claims, wherein the compound inhibits and/or reduces activation of the patient's immune system by intracellular antigens of the transplanted organ (necroinflammation).

13. The compound for use as a medicament according to any of the preceding claims, wherein administration of the compound enhances function of the transplanted organ in the patient.

14. The compound for use as a medicament according to any of the preceding claims, wherein the compound is Nec-1f,
